(19) Europäisches Patentamt

European Patent Office

Office européen des brevets

(11) EP 0 502 769 B1

(12) FASCICULE DE BREVET EUROPEEN

(45) Date de publication et mention
de la délivrance du brevet:
**17.04.1996 Bulletin 1996/16**

(51) Int. Cl.$^6$: **A61K 7/48**, A61K 7/02

(21) Numéro de dépôt: **92400527.5**

(22) Date de dépôt: **28.02.1992**

(54) **Composition cosmétique capable d'estomper les défauts de la peau**

Kosmetische Zusammensetzung zur Verminderung von Hautdefekten

Cosmetic composition capable of attenuating skin defects

(84) Etats contractants désignés:
**AT BE CH DE DK ES FR GB GR IT LI NL PT SE**

(30) Priorité: **28.02.1991 FR 9102400**

(43) Date de publication de la demande:
**09.09.1992 Bulletin 1992/37**

(73) Titulaire: **L'OREAL**
**F-75008 Paris (FR)**

(72) Inventeurs:
• **Arraudeau, Jean-Pierre**
**F-75015 Paris (FR)**
• **Mellul, Myriam**
**F-94240 l'Hay les Roses (FR)**

• **Candau, Didier**
**F-77000 Melun (FR)**

(74) Mandataire: **Tonnellier, Jean-Claude**
**Cabinet Nony & Cie.**
**29, rue Cambacérès**
**F-75008 Paris (FR)**

(56) Documents cités:
**EP-A- 0 106 762**          **DE-A- 2 612 330**
**FR-A- 2 367 486**          **FR-A- 2 373 277**
**GB-A- 2 002 652**

• **WORLD PATENTS INDEX LATEST Week 8605,
Derwent Publications Ltd., London, GB; AN 86-
033300**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention a pour objet une composition cosmétique, contenant une dispersion de poudre, capable d'estomper les défauts de la peau, tels que les microreliefs et les variations de coloration.

On a déjà proposé diverses compositions cosmétiques susceptibles d'uniformiser le teint et d'estomper le relief de la peau, c'est-à-dire de rendre moins visibles les variations de couleur et les microreliefs cutanés, les pores et les ridules. Ces compositions sont constituées de poudres dispersées dans un liant. Les poudres contiennent généralement des pigments colorés, tels que les oxydes de fer, et des charges particulaires comme le mica ou le talc, qui sont des charges sous forme lamellaire, ou encore la silice sous forme de plaquettes. L'effet revendiqué est essentiellement obtenu grâce au pouvoir couvrant apporté par les charges sous forme lamellaire ; voir par exemple le brevet US 4 839 163.

L'inconvénient de telles compositions est que l'estompage des défauts de la peau est apporté par le pouvoir couvrant des compositions, c'est-à-dire que la composition appliquée sur la peau n'est pas translucide et ne donne pas à la peau ainsi maquillée un aspect naturel. On rappelle qu'un produit de maquillage est considéré comme couvrant lorsqu'il est capable de masquer les imperfections de la peau par son opacité et/ou sa capacité à réfléchir la lumière. L'utilisation de charges de forme lamellaire a pu être considérée comme un moyen d'augmenter l'impression de transparence (voir aussi le brevet 4 839 163). Mais en fait, les charges lamellaires agissent surtout par réflexion de la lumière, et présentent pour cette raison l'inconvénient de conférer à la peau un aspect brillant peu naturel ; voir par exemple la demande de brevet publiée JP 61-69708, qui propose pour y remédier de préparer des compositions dans lesquelles on enrobe les particules de la charge avec une résine acrylique. L'inconvénient de telles compositions est que leur préparation nécessite un traitement préalable d'enrobage dont l'intégrité dans la formulation finale n'est d'ailleurs pas assurée.

On a également proposé des compositions permettant d'appliquer sur la peau un film masquant les rides, ces compositions contenant notamment un polymère filmogène hydrosoluble, un agent plastifiant et une charge particulaire telle qu'une poudre de zéolite ; voir le document PCT WO 90/04383. La couche de masquage doit en général être complétée par une couche de maquillage. La peau recevant une telle application n'a pas, ici encore, un aspect naturel.

La présente invention a pour objet un produit de maquillage qui estompe le relief de la peau, bien qu'il n'apporte pas de pouvoir couvrant notable mais donne au contraire une couche de maquillage translucide conférant à la peau maquillée un aspect naturel.

On a découvert que ce but peut être atteint par l'utilisation d'une poudre dispersée dans un liant huileux, grâce à l'utilisation d'une poudre sous la forme de particules de forme non plaquettaire, mais au contraire sphérique ou sphéroïdale, et grâce à l'utilisation de rapports volumiques liant/poudre déterminés.

Un des avantages de l'invention est que la nature du matériau particulaire constituant la charge principale de la poudre n'est pas déterminante, dès lors que le matériau est compatible avec une application sur la peau, c'est-à-dire qu'il est non toxique et non irritant, et dès lors qu'il s'agit d'un matériau donnant des particules sphériques ou sphéroïdales blanches ou faiblement colorées.

La présente invention a donc pour objet une composition cosmétique capable d'estomper les défauts de la peau tout en laissant sur celle-ci une couche translucide, ladite composition comprenant :

- une dispersion, dans un liant gras, d'une charge constituée de particules solides d'au moins un matériau compatible avec l'application sur la peau, ledit liant gras comprenant une fraction non volatile et éventuellement une fraction volatile, ladite fraction volatile représentant, lorsqu'elle est présente, moins de 20% du poids total de la composition,
- et éventuellement des ingrédients oléosolubles, dissous dans ledit liant gras, caractérisée par le fait :
- que ladite charge contient au moins 50% en poids, ou au moins 75 % en volume, par rapport au poids total de la charge, de particules sphériques ou sphéroïdales avant des dimensions de 0,5 à 50 µm,
- et que la concentration volumique C (en %) de ladite charge, dans ladite dispersion, est au moins égale à C*, étant entendu que C est égale à :

$$\frac{V}{V+V1} \times 100$$

V étant le volume de la charge, et V1 étant le volume de la fraction non volatile du liant gras contenant à l'état dissous les ingrédients oléosolubles éventuellement présents dans ladite composition, et étant entendu que C*, exprimée en %, est comprise entre 3 % et 90 % et est égale à

$$\frac{V}{V+Vh} \times 100$$

V étant le volume de la charge et Vh représentant la prise d'huile de ladite charge, ladite prise d'huile étant mesurée avec ladite fraction non volatile du liant gras contenant éventuellement, à l'état dissous, lesdits ingrédients oléosolubles, lorsqu'ils sont présents dans ladite composition.

On a en effet découvert qu'une dispersion telle que définie ci-dessus, lorsqu'elle est appliquée en couche mince sur la peau, est capable d'estomper les microreliefs et autres défauts de la peau tout en conservant à celle-ci un aspect naturel dû essentiellement au caractère translucide de cette couche mince.

Dans la définition ci-dessus, la charge totale est constituée de l'ensemble des charges particulaires, y compris les pigments éventuellement présents.

La condition donnée ci-dessus concernant la teneur de la charge en particules sphériques ou sphéroïdales est que ces particules doivent être présentes en proportion importante, généralement au moins égale à 50 % en poids de la charge. Mais lorsque les particules sphériques ou sphéroïdales sont ou comprennent des particules très légères (comme c'est le cas par exemple pour des particules creuses de polymère thermoplastique contenant un gaz), ces particules peuvent être présentes en proportion volumique importante, sans que leur proportion pondérale atteigne 50 %. Dans ce cas, la condition, pour les compositions de l'invention, est que la proportion volumique des particules sphériques ou sphéroïdales soit d'au moins 75 %, par rapport au volume total de la charge.

L'invention concerne notamment les compositions dans lesquelles la teneur pondérale de la charge en particules sphériques ou sphéroïdales est d'au moins 75 % en poids, ou d'au moins 80 % en volume, ainsi que les compositions pour lesquelles cette teneur est d'au moins 85 % en poids, ou d'au moins 90 % en volume.

Il est connu que la prise d'huile d'une poudre varie non seulement avec le matériau constituant la poudre, mais aussi avec la nature du constituant huileux. Bien entendu, pour la détermination de la concentration volumique C de la charge, ainsi que pour la détermination de C*, on utilise le liant réel de la composition finale, c'est-à-dire non seulement la fraction non volatile du liant gras proprement dit, mais aussi les ingrédients oléosolubles éventuellement présents (par exemple, les agents tensioactifs lorsqu'ils sont présents) dans la composition cosmétique finale.

Le plus souvent, on utilisera des charges telles que la concentration volumique C* est comprise entre 4 et 80 %.

Dans la dispersion de l'invention, la concentration volumique C de la charge, calculée comme indiqué précédemment, est de préférence telle que le rapport de cette concentration C à la concentration C* soit superieure à 1, et en particulier supérieure ou égale à 1,1.

On rappelle que la prise d'huile est mesurée en déterminant le volume Vh de la fraction non volatile du liant gras juste nécessaire pour combler les interstices entre les particules de la poudre (charge). La prise d'huile peut être mesurée par exemple selon la norme américaine ASTM D281-84.

Soit V le volume de la charge et V1 le volume de la fraction non volatile du liant gras utilisé dans une dispersion donnée.

La concentration C* (en %) est égale à :

$$\frac{V}{V+Vh} \times 100$$

La concentration volumique C de la charge (en %), qui selon l'invention doit être au moins égale à C*, dans la dispersion considérée, est égale à :

$$\frac{V}{V+V1} \times 100$$

On a découvert que le caractère translucide de la dispersion telle que définie ci-dessus augmente lorsque C augmente, pour les valeurs de C supérieures à C*. Il est donc possible de régler à volonté, dans une certaine mesure, le caractère translucide de la dispersion, et donc du maquillage obtenu avec ladite dispersion. Généralement, on choisira C de façon que la transmission de la lumière, mesurée entre deux lames de quartz sur un trajet optique de 100 μm, de ladite dispersion (contenant tous les ingrédients de cette dispersion à l'exception des huiles volatiles éventuellement présentes), soit au moins égale à 5 %, à une longueur d'onde de 560 nm. Le plus souvent, on obtient de bons résultats de maquillage lorsque ladite transmission est comprise entre 5 et 30 % environ.

De préférence, les particules sphériques ou sphéroïdales ont des dimensions allant de 0,5 à 25 μm, et en particulier de 1 à 15 μm. Ces dimensions peuvent être déterminées par diffusion quasi-élastique de la lumière à partir de la distribution en volume.

D'une façon générale, la préparation de poudres sous forme de particules sphériques ou sphéroïdales est connue. En outre, il existe déjà dans le commerce de telles poudres. On peut citer, à titre d'exemples non limitatifs, des poudres minérales telles que la silice sphérique, les dioxydes de titane sphériques comme le Sphérititan (marque déposée), les billes de verre et de céramique commercialisées par la Société 3M sous les dénominations MACROLITES ; des poudres de matériaux organiques d'origine naturelle comme les amidons de maïs, de blé, de riz, réticulés ou non ; des poudres de polymères de synthèse (éventuellement réticulés) sphéronisées, comme des poudres de polyamides (par exemple de nylon ou de poly-bêta-alanine), de polyéthylène, d'acides poly-méthacryliques, de polystyrène (réticulé par le divinylbenzène), de résine de silicone, de téflon (par exemple Fluon, particules commercialisées par Montefluos, Hostaflon, particules commercialisées par Hoechst), etc.

On peut également utiliser des poudres en matériau synthétique thermoplastique sous forme de microsphères creuses, qui peuvent être obtenues notamment selon les procédés connus décrits par exemple dans le brevet US-3615972 et la demande de brevet EP-56219. La partie creuse de ces microsphères contient un gaz tel qu'un hydrocarbure, de l'air ou tout autre gaz approprié. Les microsphères creuses peuvent être réalisées en tout matériau thermoplastique non toxique et non irritant pour la peau, par exemple des polymères ou copolymères de dérivés éthyléniques (en particulier le polyéthylène, le polystyrène, les copolymères chlorure de vinyle-acrylonitrile), des polyamides, des polyesters, des polymères urée-formaldéhyde, ou des copolymères de chlorure de vinylidène (en particulier des copolymères de chlorure de vinylidène et d'acrylonitrile). Ces microsphères creuses sont très légères : leur masse spécifiques peut être par exemple de l'ordre de 0,01 à 0,1 g/cm$^3$.

Parmi les liants, on peut citer notamment les huiles animales, végétales ou synthétiques, les mélanges d'huile(s) et de cire(s) et en particulier l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide, etc... ; des huiles d'hydrocarbures, telles que des huiles de paraffine, le squalane, la vaseline, etc ; des esters tels que le myristate d'isopropyle, le palmitate d'isopropyle, le stéarate de butyle, le laurate d'hexyle, l'isononanate d'isononyle, le palmitate de 2-éthyl-hexyle, le laurate de 2-hexyl-décyle, le palmitate de 2-octyl-décyle, le myristate de 2-octyl-dodécyle, le succinate de 2-di-éthyl-hexyle, le malate de diisostéaryle, le lactate de 2-octyl-dodécyle, le triisostéarate de glycérine, le triisostéarate de diglycérine, etc... ; des huiles de silicone comme les poly-méthylsiloxanes, les poly-méthylphénylsiloxanes, des polysiloxanes modifiés par des acides gras, des polysiloxanes modifiés par des alcools gras, des polysiloxanes modifiés par des polyoxyalkylènes, des silicones fluorées, etc... ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, etc ... ; des alcools gras supérieurs tels que le cétanol, l'alcool stéarylique, l'alcool oléique, etc ... ; les cires peuvent être choisies notamment parmi la cire de carnauba, la cire de candelilla, la cire d'abeille, la cire de baleine, des lanolines, des cires microcristallines, etc...

Le liant gras peut contenir en outre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 0,5.10$^2$Pa).

Les huiles volatiles lorsqu'elles sont présentes, représentent généralement moins de 20 %, et en particulier moins de 10 % en poids de la composition finale.

Parmi les huiles volatiles pouvant être présentes comme agents d'étalement dans la composition de l'invention, on citera par exemple des huiles de silicone telles que l'hexaméthyldisiloxane, le cyclopentadiméthylsiloxane, le cyclotétraméthylsiloxane, des huiles fluorées comme celle commercialisée sous la dénomination Galden (Montefluos) ou des huiles isoparaffiniques telles que celles commercialisées sous la dénomination ISOPAR (E, G, L ou H).

De préférence, le liant gras est tel que son constituant, ou le mélange de ses constituants autres que les huiles volatiles, mais y compris les autres ingrédients oléosolubles de la composition finale, a une température de fusion ou de ramollissement non supérieure à 37°C, en particulier non supérieure à 32°C.

La fraction non volatile du liant gras est de préférence telle qu'elle présente une transmission de la lumière, à 560 nm, d'au moins 90 % sur un trajet optique de 1 cm.

La composition de l'invention peut également contenir, outre la charge principale (essentiellement constituée de particules sphéroïdales, comme indiqué ci-dessus), des pigments blancs ou colorés habituellement utilisés dans les compositions de ce type. Ces pigments blancs ou colorés sont incorporés dans la dispersion décrite ci-dessus. Ils ont de préférence des dimensions de particules inférieures à 1 μm, et en particulier inférieures à 0,5 μm pouvant aller par exemple de 0,02 à 0,3 μm.

Les pigments colorés sont notamment des oxydes métalliques tels que des oxydes fer (jaune, rouge et/ou noir) ou de chrome ; d'autres dérivés métalliques, comme par exemple le nitrure de titane ; ou des pigments organiques, par exemples des pigments mélaniques (noir de seiche).

Parmi les pigments blancs, on citera les oxydes de titane, de zinc et de zirconium, ou encore le sulfate de baryum. Ces pigments blancs peuvent être utilisés notamment en combinaison avec les pigments colorés pour donner de la consistance à la couleur.

En outre, on peut remplacer tout ou partie des pigments colorés (ayant leur couleur propre) par des particules colorées dans la masse.

Les pigments ou les particules colorées peuvent être introduits dans la dispersion de l'invention soit isolément, soit déposés sur des particules faisant partie de la charge principale de la dispersion.

Lorsque des pigments blancs ou colorés sont incorporés dans la dispersion, ils sont pris en compte dans la détermination de la concentration volumique de la charge et dans la détermination de C*.

Les pigments blancs et/ou colorés, lorsqu'ils sont présents, représentent moins de 25 % en poids, et généralement moins de 15 % en poids, de la charge totale.

De préférence, la charge principale (c'est-à-dire autre que les pigments) ne contient pas de particules de forme lamellaire. L'invention s'étend toutefois à des compositions telles que définies ci-dessus contenant en outre une proportion minoritaire de particules lamellaires (par exemple de talc, de mica, de séricite ou de silice), cette proportion étant

4

par exemple telle que la transmission de la lumière de la dispersion obtenue reste au moins égale à 5 %, cette transmission étant mesurée dans les conditions déjà mentionnées plus haut.

La dispersion telle que définie ci-dessus peut être incorporée dans un support cosmétique de façon à constituer une composition sous forme d'une émulsion simple ou triple eau-dans-l'huile ou huile-dans-l'eau, ou bien d'un gel.

La dispersion peut également constituer à elle seule la composition de l'invention qui se présente alors sous la forme d'un fard anhydre comme une crème teintée coulée, un fard à paupières ou de façon générale sous toutes les formes usuelles de produits de maquillage constitués de dispersions solides/liquides.

Le support cosmétique pourra comprendre notamment des tensioactifs, non ioniques, anioniques, cationiques ou amphotères. On peut citer par exemple les sucres et les esters d'acides gras des sucres, comme les esters de sucrose, de mannitol ou de sorbitan ; les esters d'acides gras de la glycérine ou de la polyglycérine ; les esters d'acides gras du propylèneglycol ; les esters d'acides gras du sorbitan polyoxyéthyléné ; les esters d'acides gras du sorbitol polyoxyéthyléné ; les esters d'acides gras de la glycérine polyoxyéthylénée ; les esters d'acides gras du polyéthylèneglycol ; les alkyl-éthers polyoxyéthylénés ; les phytostérols polyoxyéthylénés, les polyoxypropylène-alkyléthers polyoxyéthylénés ; les alkyl-phényl éthers polyoxyéthylénés ; les éthers du cholestanol polyoxyéthylénés ; les dérivés de lanoline polyoxyéthylénés ; les alkylamines polyoxyéthylénées ; les alkylamides polyoxyéhylénés.

La composition de l'invention peut également contenir divers ingrédients actifs ou additifs usuels, non particulaires, tels que les vitamines et leurs dérivés ; les filtres anti-solaires, les parfums, etc. Pour favoriser la stabilité du système ou produire un effet filmogène supplémentaire, on peut également ajouter un colloïde tel qu'un polymère habituellement utilisé dans les produits cosmétiques, comme par exemple les acides poly-acryliques et poly-méthacryliques ainsi que leurs dérivés, notamment leurs esters, et les copolymères correspondants ; les polyquaterniums ; les gommes et les polymères de sucres comme les celluloses et leurs dérivés ; les poly-vinylpyrrolidones et leurs copolymères ; des agents d'étalement tels que les huiles volatiles déjà citées, etc.

Les compositions de l'invention contiennent généralement :

- de 10 à 100 % en poids de ladite dispersion ;
- de 0 à 90 % en poids d'eau ;
- de 0 à 10 % en poids de tensioactifs ;
- de 0 à 40 % en poids d'autres ingrédients actifs ou additifs usuels non particulaires.

On considère ici que les ingrédients non particulaires sont ceux qui se trouvent dans un état suffisamment divisé pour avoir des dimensions inférieures à 0,05 μm, et en particulier inférieures à 0,02 μm.

Les cosmétiques ainsi obtenus sont non couvrants ou n'ont qu'un faible pouvoir couvrant, sont translucides, et permettent d'obtenir un effet d'estompage et de lissage de la peau qui rend moins visibles le grain de la peau, les pores et autres petites rides tout en conservant un aspect naturel au maquillage.

L'invention a également pour objet l'utilisation, dans la préparation d'une composition cosmétique capable d'estomper les défauts de la peau tout en laissant sur celle-ci une couche translucide, d'une dispersion, dans un liant gras, d'une charge constituée de particules solides d'au moins un matériau compatible avec l'application sur la peau, ladite charge contenant au moins 50 % en poids, ou au moins 75 % en volume, par rapport à la charge totale, de particules sphériques ou sphéroïdales ayant des dimensions de 0,5 à 50 μm, ladite charge ayant une prise d'huile telle que sa concentration volumique $C^*$, quand le volume du liant est égal à celui de la prise d'huile, est comprise entre 3 et 90 %, et la concentration volumique de la charge, dans ladite dispersion, calculée sans tenir compte des huiles volatiles éventuellement présentes dans le liant gras, étant au moins egale à $C^*$.

La dispersion utilisée peut présenter les autres caractéristiques déjà décrites ci-dessus.

L'invention a en outre pour objet un procédé de préparation d'une composition telle que définie ci-dessus, ce procédé étant caractérisé par le fait que, pour préparer ladite dispersion, l'on choisit une charge de particules solides d'au moins un matériau compatible avec l'application sur la peau, ladite charge contenant au moins 50 % en poids, ou au moins 75 % en volume, et en particulier au moins 85 % en poids, par rapport à la charge totale, de particules solides sphériques ou sphéroïdales ayant des dimensions de 0,5 à 50 μm, ladite charge ayant une prise d'huile telle que sa concentration volumique $C^*$, quand le volume du liant est égal à celui de la prise d'huile, est comprise entre 3 et 90 %, et par le fait que l'on choisit la concentration volumique de la charge, dans ladite dispersion, mesurée sans tenir compte des huiles volatiles éventuellement présentes dans le liant gras, de façon qu'elle soit au moins égale à $C^*$.

La dispersion obtenue est ensuite mise sous forme appropriée, selon les méthodes usuelles (additions d'ingrédients actifs usuels et/ou mise sous forme d'émulsion et/ou conditionnement en conteneurs, etc...), pour obtenir la composition cosmétique finale désirée.

Les compositions de l'invention sont appliquées sur la peau de la manière usuelle. Après l'application, l'eau de la composition et les huiles volatiles éventuellement présentes, s'évaporent pour laisser sur la peau une couche mince constituée principalement par la charge de particules sphériques, les pigments (s'ils sont présents), et la fraction non volatile du liant gras.

L'invention a également pour objet un procédé de traitement cosmétique, permettant d'estomper chez les humains les défauts de la peau, notamment de la peau du visage et du cou, ce procédé étant caractérisé par le fait que l'on applique sur les zones de peau à traiter une quantité efficace d'une composition telle que définie précédemment. Il est évident que les quantités efficaces peuvent être déterminées aisément par chaque utilisateur.

Les exemples suivants illustrent l'invention sans toutefois la limiter.

EXEMPLE 1 : Fond de teint fluide On réalise une dispersion de silice sphérique, de poudre de nylon, de poudre d'amidon et de pigments, dans les proportions relatives indiquées ci-dessous, dans un liant gras de façon que la concentration volumique des particules dans le liant soit égale à 80 %.

La concentration volumique C* de la charge est 65 %.

On incorpore cette dispersion dans de l'eau pour obtenir, sous forme d'une émulsion de type huile-dans-l'eau, une composition ayant la formule suivante :

| Charges : | |
|---|---|
| Oxyde de fer jaune | 0,43 % en poids |
| Oxyde de fer rouge | 0,21 % en poids |
| Oxyde de fer noir | 0,11 % en poids |
| Oxyde de titane rutile | 3,25 % en poids |
| Silice sphérique | 13,63 % en poids |
| Poudre de nylon | 6,78 % en poids |
| Poudre d'amidon | 11,44 % en poids |

| Phase aqueuse : | |
|---|---|
| Eau | 54,40 % en poids |
| Méthyl parabène (conservateur) | 0,30 % en poids |

| Liant gras : | |
|---|---|
| Poly(diméthylsiloxane) | 2,22 % en poids |
| Poly(isobutylène) hydrogéné | 1,92 % en poids |
| Alcool stéarylique POE (2) | 2,50 % en poids |
| Alcool stéarylique POE (21) | 2,50 % en poids |
| Propylparabène (conservateur) | 0,30 % en poids |

La silice sphérique utilisée est celle commercialisée sous la marque SILICA BEADS SB 150 par MAPRECOS (dimensions : 15 µm).

La poudre de nylon utilisée est celle commercialisée sous la marque ORGASOL par ATO (dimensions : 14,5 µm).

La poudre d'amidon utilisée est celle commercialisée sous la marque DRYFLO par NATIONAL STARCH (dimensions : 16 µm).

Le poly (diméthylsiloxane) est celui commercialisé sous la marque DC 200 FLUID 350 et par DOW CORNING.

Le poly(isobutylène) hydrogéné est celui commercialisé sous la marque PARLEAM par NICHIYU.

Les alcools stéaryliques POE (2) et POE (21) sont commercialisés par ICI.

On obtient un produit de maquillage de type fond de teint applicable sur le visage et le cou, par exemple avec une éponge en latex.

Lors d'un essai, non public, d'application de la composition de cet exemple 1, organisé auprès d'un panel d'utilisatrices, celles-ci ont unanimement déclaré que le pouvoir couvrant est faible et que le teint est unifié ; les cernes, les taches de la peau, et éventuellement les couperoses sont atténués ; la peau semble lissée ; le grain de la peau est affiné. Le maquillage est très naturel.

EXEMPLE 2 : Crème pour le contour des yeux

De façon analogue, on a préparé une composition sous la forme d'une émulsion huile-dans l'eau, ayant la formulation suivante :

| | |
|---|---|
| Tospearl 3120 | 16,73 |
| Silice SB 150 | 18,68 |
| Eau | 49,900 |
| Parahydroxybenzoate de méthyle | 0,300 |
| Alcool stéarylique | 0,600 |
| Alcocl cétylique | 0,600 |
| Lanoline liquide | 0,580 |
| Acide stéarique | 0,290 |
| Squalane | 2,520 |
| Stéarate de POE(20) | 2,000 |
| Mono et distéarate de glycérol auto-émulsionnant | 2,000 |
| Parahydroxybenzoate de propyle | 0,300 |
| Copolymère acrylate d'ammonium/acrylamide (émulsion aqueuse à 9 % en poids) * | 5,500 |

C* = 60 %.
La concentration volumique de la poudre dans le liant est de 80 %.
Tospearl 3120 est une marque déposée pour une poudre de silicone commercialisée par TOSHIBA (dimensions : 12 $\mu$m).
Le stéarate de POE (20) est le tensioactif commercial Mirj 49 de ICI
Le mélange de mono- et distéarate de glycérol auto-émulsionnant est le produit commercial ARLACEL 165 de ICI.

*L'émulsion aqueuse est commercialisée par Hoechst.

EXEMPLE 3 : Crème de soin "contour des yeux"

De façon analogue, on a préparé une composition sous la forme d'une émulsion huile-dans l'eau, ayant la formulation suivante :

| | |
|---|---|
| Tospearl 3120 | 34,630 |
| Eau | 45,900 |
| Parahydroxybenzoate de méthyle | 0,300 |
| Glycérine | 2,000 |
| Polyglycérine 500 | 2,000 |
| Alcool stéarylique | 0,600 |
| Alcool cétylique | 0,600 |
| Lanoline liquide | 0,710 |
| Squalane | 3,100 |
| Acide stéarique | 0,360 |
| Stéarate de POE(20) | 2,000 |
| Mono et distéarate de glycérol | 2,000 |
| Parahydroxybenzoate de propyle | 0,300 |
| Copolymère acrylate d'ammonium/acrylamide (émulsion aqueuse ; Hoechst) | 5,500 |
| C* = 60 %<br>La concentration volumique de la charge dans le liant est de 80 %.<br>La polyglycérine 500 est commercialisée par ROSSOW | |

EXEMPLE 4 : Crème de soin non teintée pour le visage

De façon analogue, on a préparé une composition sous la forme d'une émulsion huile-dans l'eau, ayant la formulation suivante :

| | |
|---|---|
| Silice SB 700 | 23,750 |
| Eau | 47,900 |
| Parahydroxybenzoate de méthyle | 0,300 |
| Alcool stéarylique | 0,600 |
| Alcool cétylique | 0,600 |
| Lanoline liquide | 2,580 |
| Squalane | 11,180 |
| Acide stéarique | 1,290 |
| Stéarate de POE (20) | 3,000 |
| Mono et distéarate de glycérol | 3,000 |
| Parahydroxybenzoate de propyle | 0,300 |
| Copolymère acrylate d'ammonium/acrylamide (émulsion aqueuse ; Hoechst) | 5,500 |

C* = 62 %.

La concentration volumique de la charge dans le liant est de 70 %.

EXEMPLE 5 : Crème non teintée pour le contour des yeux

De façon analogue, on a préparé une composition sous la forme d'une émulsion huile-dans l'eau, ayant la formulation suivante :

| | |
|---|---|
| Silice SB 700 | 28,888 |
| Eau | 49,900 |
| Parahydroxybenzoate de méthyle | 0,300 |
| Alcool stéarylique | 0,600 |
| Alcool cétylique | 0,600 |
| Lanoline liquide | 1,700 |
| Squalane | 7,364 |
| Acide stéarique | 0,848 |
| Stéarate de POE (20) | 2,000 |
| Mono et distéarate de glycérol | 2,000 |
| Parahydroxybenzoate de propyle | 0,300 |
| Copolymère acrylate d'ammonium/acrylamide (émulsion aqueuse ; Hoechst) | 5,500 |

$C^* = 60$ %.
La concentration volumique de la charge dans le liant est de 80 %.
La silice SB 700 est une poudre de silice commercialisée par NAPRECOS (dimensions : 16 µm).

EXEMPLE 6 : Crème de soin non teintée

Une dispersion de silice dans le liant (concentration volumique de la silice : 75 %) est réalisée, puis incorporée dans une émulsion eau-dans-l'huile :

| | |
|---|---|
| Silice SB 700 | 26,240 |
| Eau | 53,400 |
| Parahydroxybenzoate de méthyle | 0,300 |
| Alcool stéarylique | 0,600 |
| Alcool cétylique | 0,600 |
| Lanoline liquide | 2,152 |
| Squalane | 9,332 |
| Acide stéarique | 1,076 |
| Copolymère dodécylglycol/Méthoxypolyéthylèneglycol (220E) | 2,500 |
| Sesquioléate de polyglycéryle | 3,500 |
| Parahydroxybenzoate de propyle | 0,300 |

$C^* = 60$ %.
Le copolymère dodécylglycol/méthoxypolyéthylèneglycol (220E) est commercialisé par AKZO (Elfacos E200).

EXEMPLE 7 : Crème non teintée pour le contour des yeux

De façon analogue, on a préparé une composition sous la forme d'une émulsion huile-dans l'eau, ayant la formulation suivante :

| | |
|---|---|
| Silice SB 150 | 36,144 |
| Eau | 49,900 |
| Parahydroxybenzoate de méthyle | 0,300 |
| Alcool stéarylique | 0,600 |
| Alcool cétylique | 0,600 |
| Lanoline liquide | 0,456 |
| Squalane | 1,972 |
| Acide stéarique | 0,228 |
| Stéarate de POE (20) | 2,000 |
| Mono et distéarate de glycérol | 2,000 |
| Parahydroxybenzoate de propyle | 0,300 |
| Copolymère acrylate d'ammonium/acrylamide (émulsion aqueuse ; Hoechst) | 5,500 |
| $C^* = 41$ %. La concentration volumique de la charge dans le liant est de 80 %. | |

EXEMPLE 8 : Crème de soin non teintée pour le visage

De façon analogue, on a préparé une composition sous la forme d'une émulsion huile-dans l'eau, ayant la formulation suivante :

| | |
|---|---|
| Silice SB 150 | 31,840 |
| Eau | 49,900 |
| Parahydroxybenzoate de méthyle | 0,300 |
| Alcool stéarylique | 0,600 |
| Alcool cétylique | 0,600 |
| Lanoline liquide | 1,192 |
| Squalane | 5,172 |
| Acide stéarique | 0,596 |
| Stéarate de POE (20) | 2,000 |
| Mono et distéarate de glycérol | 2,000 |
| Parahydroxybenzoate de propyle | 0,300 |
| Copolymère acrylate d'ammonium/acrylamide (émulsion aqueuse ; Hoechst) | 5,500 |
| $C^* = 41$ %. La concentration volumique de la charge dans le liant est de 60 %. | |

EXEMPLE 9 : <u>Crème fluide non teintée, pour le visage</u>

De façon analogue, on a préparé une composition sous la forme d'une émulsion huile-dans l'eau, ayant la formulation suivante :

| | |
|---|---|
| Silice SB 150 | 17,384 |
| Tospearl 3120 | 15,588 |
| Eau | 42,90 |
| Parahydroxybenzoate de méthyle | 0,30 |
| Alcool cétylique | 0,60 |
| Alcool stéarylique | 0,60 |
| Lanoline liquide | 1,004 |
| Acide stéarique | 0,50 |
| Squalane | 4,344 |
| Stéarate de POE(20) | 2,00 |
| Mono et distéarate de glycérol auto-émulsionnable | 2,00 |
| Parahydroxybenzoate de propyle | 0,30 |
| Copolymère acrylate d'ammonium/acrylamide (émulsion aqueuse ; Hoechst) | 5,50 |
| Copolymère acrylamide/chlorure de diméthyldiallyl ammonium* | 2,00 |
| Cyclopenta dimethylsiloxane | 5,00 |
| C* = 52 %<br>Concentration de la charge dans le liant : C = 70 % | |

*commercialisé par la Société MERCK sous la dénomination Merquat S.

EXEMPLE 10 : Crème légère non teintée pour le visage

De façon analogue, on a préparé une composition sous la forme d'une émulsion huile-dans l'eau, ayant la formulation suivante :

| | |
|---|---|
| Silice SB 150 | 18,68 |
| Tospearl 3120 | 16,73 |
| Eau | 42,98 |
| Parahydroxybenzoate de méthyle | 0,30 |
| Alcool cétylique | 0,60 |
| Alcool stéarylique | 0,60 |
| Lanoline liquide | 0,50 |
| Acide stéarique | 0,29 |
| Squalane | 2,52 |
| Stéarate de POE(20) | 2,00 |
| Mono et distéarate de glycérol auto-émulsionnable | 2,00 |
| Parahydroxybenzoate de propyle | 0,30 |
| Perfluoropolyéther* | 2,00 |
| Copolymère acrylate d'ammonium/acrylamide (solution aqueuse ; Hoechst) | 5,50 |
| Cyclopentadiméthylsiloxane | 5,00 |
| C* = 52 %<br>C = 80 % | |

*L'huile perfluoropolyéther est commercialisée par la Société Montefluos sous la dénomination Fomblin HC/R.

EXEMPLE 11 : Crème teintée peur le visage

De façon analogue, on a préparé une composition sous la forme d'une émulsion huile-dans l'eau, ayant la formulation suivante :

| | |
|---|---|
| Oxyde de fer jaune | 0,13 |
| Oxyde de fer rouge | 0,06 |
| Oxyde de fer noir | 0,03 |
| Dioxyde de titane | 0,665 |
| Silice SB 150 | 17,92 |
| Acrylate/C10-C30 alkyl acrylate crosspolymer | 2,00 |
| Polymère carboxyvinylique | 2,00 |
| Polyglycérine 500 | 2,00 |
| Glycérine | 2,00 |
| Parahydroxybenzoate de méthyle | 0,30 |
| Perfluoropolyéther | 2,00 |
| Eau | 48,17 |
| Métaphosphate de sodium | 0,30 |
| Alcool cétylique | 0,60 |
| Alcool stéarylique | 0,60 |
| Lanoline liquide | 0,564 |
| Acide stéarique | 0,281 |
| Squalane | 2,441 |
| Parahydroxybenzoate de propyle | 0,30 |
| Tospearl 3120 | 16,044 |
| Dioxyde de titane micronisé | 0,665 |
| Triéthanolamine | 0,930 |

$C^* = 52\ \%$
$C = 80\ \%$
Le polymère acrylate/C10-C30 alkyl acrylate crosspolymer est commercialisé par la Société Goodrich sous la dénomination Pemulen TR2.
L'huile perfluoropolyéther est commercialisée par la Société Montefluos sous la dénomination Fomblin HC/R.
Dans le présent exemple, et dans les exemples suivants, le dioxyde de titane a, sauf indications contraires, des dimensions de particules de 0,2 à 0,3 $\mu$m.

EXEMPLE 12 : Fond de teint fluide

De façon analogue, on a préparé une conposition sous la forme d'une émulsion huile-dans l'eau, ayant la formulation suivante :

| | |
|---|---|
| Oxyde de fer jaune | 0,60 |
| Oxyde de fer rouge | 0,39 |
| Oxyde de fer noir | 0,11 |
| Dioxyde de titane | 2,90 |
| Silice SB 150 | 21,904 |
| Eau | 40,50 |
| Polyglycérine 500 | 3,50 |
| Parahydroxybenzoate de méthyle | 0,20 |
| Perfluoropolyéther | 2,00 |
| Huile de rosier muscat | 0,276 |
| Huile de palme | 0,344 |
| Huile Parleam | 0,704 |
| Huile de jojoba | 2,112 |
| Mono et distéarate de glycéryle | 4,00 |
| Monostéarate de glycéryle oxyéthyléné (30 0E) | 4,00 |
| Parahydroxybenzoate de propyle | 0,20 |
| Poudre de nylon | 10,660 |
| Eau | 5,00 |
| Parahydroxybenzoate de méthyle | 0,30 |
| Parfum | 0,30 |

C* = 43 %
C = 80 %
Le mono et distéarate de glycéryle est commercialisé par la Société Goldschmidt sous la dénomination TeginM.
Le monostéarate de glycéryle oxyéthyléné (30 OE) est commercialisé par la Société Goldschmidt sous la dénomination de Tagat S.

EXEMPLE 13 : Fond de teint

De façon analogue, on a préparé une composition sous la forme d'une émulsion huile-dans l'eau, ayant la formulation suivante :

| | |
|---|---|
| Oxyde de fer jaune | 0,60 |
| Oxyde de fer rouge | 0,39 |
| Oxyde de fer noir | 0,11 |
| Dioxyde de titane | 2,90 |
| Silice SB 150 | 20,496 |
| Eau | 30,60 |
| Polyglycérine 500 | 3,00 |
| Glycérine | 3,00 |
| Parahydroxybenzoate de méthyle | 0,20 |
| Perfluoropolyéther | 2,00 |
| Huile de rosier muscat | 0,444 |
| Huile de palme | 0,552 |
| Huile Parleam | 0,136 |
| Huile de jojoba | 3,40 |
| Stéarate de POE(20) | 2,00 |
| Mono et distéarate de glycérol auto-émulsionnable | 2,00 |
| Acétate de tocophéryle | 0,50 |
| Parahydroxybenzoate de propyle | 0,20 |
| Dioxyde de titane micronisé | 1,00 |
| Poudre de nylon | 9,972 |
| Copolymère acrylate d'ammonium/acrylamide (émulsion aqueuse à 6 %) | 16,00 |
| Parahydroxybenzoate de méthyle | 0,30 |
| Parfum | 0,20 |

C* = 43 %
C = 70 %
Le dioxyde de titane micronisé est commercialisé par TAYCA (dimension moyenne des particules : 0,015 μm). Il joue ici le rôle de filtre solaire, absorbant notamment l'ultraviolet.
La poudre de nylon est commercialisée par ATOCHEM sous la dénomination ORGASOL (dimension moyenne des particules : 14,5 μm).
L'huile dite de rosier muscat est un mélange commercial de triglycérides d'acides oléique, linoléique et linolénique.

Exemple 14 : Fond de teint

On a préparé une composition de fond de teint ayant la formulation suivante :

| | |
|---|---|
| Pigments colorés (oxydes de fer) | 0,6 |
| Dioxyde de titale | 2,4 |
| Silice | 10,0 |
| Talc | 15,0 |
| Silicate de magnésium et d'aluminium (poudre) | 0,75 |
| Expancel DE 550 | 0,3 |
| Eau | 34,6 |
| Triéthanolamine | 1,1 |
| Acide stéarique | 2,2 |
| Glycérine | 3,0 |
| Stéarate de glycérine | 2,2 |
| Diméthicone Cyclomethicone Filtre UV (méthoxycinnamate d'octyle) | 1,0 |
| Conservateurs | 0,65 |
| Parfum | 0,2 |

La silice utilisée est celle commercialisée sous la dénomination Silicabeads SB (Myoshi).
Diméthicone est un polydiméthylsiloxane commercialisé sous la dénomination Dow Corning 200 Fluid 10 cs.
Cyclomethicone est une huile de silicone volatile commercialisée sous la dénomination Dow Corning 245 Fluid.
Expancel DE 550 est la marque désignant une poudre de microsphères creuses commercialisée par Kemanord Plast.

**Revendications**

1.  Composition cosmétique capable d'estomper les défauts de la peau tout en laissant sur celle-ci une couche translucide, ladite composition comprenant :

    - une dispersion, dans un liant gras, d'une charge constituée de particules solides d'au moins un matériau compatible avec l'application sur la peau, ledit liant gras comprenant une fraction non volatile et éventuellement une fraction volatile, ladite fraction volatile représentant, lorsqu'elle est présente, moins de 20 % du poids total de la composition,
    - et éventuellement des ingrédients oléosolubles, dissous dans ledit liant gras, caractérisée par le fait :
    - que ladite charge contient au moins 50 % en poids, ou au moins 75 % en volume, par rapport au poids total de la charge, de particules sphériques ou sphéroïdales ayant des dimensions de 0,5 à 50 μm,
    - et que la concentration volumique C (en %) de ladite charge, dans ladite dispersion, est au moins égale à C\*, étant entendu que C est égale à :

    $$\frac{V}{V+V1} \times 100$$

    V étant le volume de la charge, et V1 étant le volume de la fraction non volatile du liant gras contenant à l'état dissous les ingrédients oléosolubles éventuellement présents dans ladite composition,
    et étant entendu que C\*, exprimée en %, est comprise entre 3 % et 90 % et est égale à :

    $$\frac{V}{V+Vh} \times 100$$

    V étant le volume de la charge et Vh représentant la prise d'huile de ladite charge, ladite prise d'huile étant mesurée avec ladite fraction non volatile du liant gras contenant éventuellement, à l'état dissous, lesdits ingrédients oléosolubles, lorsqu'ils sont présents dans ladite composition.

2. Composition selon la revendication 1, caractérisée par le fait que ladite charge contient au moins 75 % en poids, ou au moins 80 % en volume desdites particules sphériques ou sphéroïdales, par rapport à la charge totale.

3. Composition selon la revendication 1, caractérisée par le fait que ladite charge contient au moins 85 % en poids, ou au moins 90 % en volume, desdites particules sphériques ou sphéroïdales, par rapport à la charge totale.

4. Composition selon l'une quelconque des revendications précédentes, caractérisée par le fait que lesdites particules sphériques ou sphéroïdales ont des dimension de 0,5 à 25 μm.

5. Composition selon la revendication précédente 1, caractérisée par le fait que lesdites particules sphériques ou sphéroïdales ont des dimensions pouvant aller de 1 à 15 μm.

6. Composition selon l'une quelconque des revendications précédentes, caractérisée par le fait que ladite concentration volumique C* est comprise entre 4 et 80 %.

7. Composition selon l'une quelconque des revendications précédentes, caractérisée par le fait que le rapport de ladite concentration volumique C à la concentration C* est supérieur à 1.

8. Composition selon la revendication précédente, caractérisée par le fait que ledit rapport est supérieur ou égal à 1,1.

9. Composition selon la revendication précédente, caractérisée par le fait que ledit rapport est suffisamment élevé pour que la transmission de la lumière, mesurée entre deux lames de quartz sur un trajet optique de 100 μm, de ladite dispersion (contenant tous les ingrédients de cette dispersion à l'exception des huiles volatiles éventuellement présentes), soit au moins égale à 5 %, à une longueur d'onde de 560 nm.

10. Composition selon l'une quelconque des revendications précédentes, caractérisée par le fait que ladite charge, à l'exception des pigments éventuellement présents, est constituée uniquement desdites particules sphériques ou sphéroïdales.

11. Composition selon l'une quelconque des revendications 1 à 6, caractérisée par le fait que ladite charge contient en outre une quantité minoritaire de particules de forme lamellaire.

12. Composition selon l'une quelconque des revendications précédentes, caractérisée par le fait que ledit matériau est un matériau minéral, un matériau organique d'origine naturelle, ou un polymère de synthèse.

13. Composition selon l'une quelconque des revendications précédentes, caractérisée par le fait que ledit matériau est choisi parmi la silice, le dioxyde de titane, les résines de silicone, l'amidon, les polyamides, le polyéthylène, les acides polyméthacryliques, le polystyrène éventuellement réticulé, la poly-bêta-alanine et le polytétrafluoroéthylène.

14. Composition selon l'une quelconque des revendications précédentes, caractérisée par le fait que ladite charge comprend en outre des pigments blancs ou colorés.

15. Composition selon la revendication précédente, caractérisée par le fait que lesdits pigments blancs ou colorés ont des dimensions de particules inférieures à 1 μm, et en particulier inférieures à 0,5 μm.

16. Composition selon l'une quelconque des revendications 14 et 15, caractérisée par le fait que lesdits pigments blancs ou colorés sont présents à raison de moins de 25 % en poids, et en particulier moins de 15 %, par rapport à la charge totale.

17. Composition selon l'une quelconque des revendications 1 à 13, caractérisée par le fait qu'elle est exempte de pigments blancs ou colorés.

18. Composition selon l'une quelconque des revendications précédentes, caractérisée par le fait que le liant est une huile animale, végétale ou synthétique, un mélange d'huiles ou un mélange d'huile(s) et de cire(s).

19. Composition selon l'une quelconque des revendications précédentes, caractérisée par le fait que la fraction non volatile dudit liant gras a un point de fusion ou de ramollissement non supérieur à 37 °C.

20. Composition selon l'une quelconque des revendications précédentes, caractérisée par le fait que la fraction non volatile dudit liant gras a un point de fusion ou de ramollissement non supérieur à 32°C.

21. Composition selon l'une quelconque des revendications précédentes, caractérisée par le fait que la fraction non volatile du liant gras présente une transmission de la lumière à 560 nm d'au moins 90 % sur un trajet optique de 1 cm.

22. Composition selon l'une quelconque des revendications précédentes, caractérisée par le fait que ladite dispersion est incorporée dans un support cosmétique de façon à constituer une émulsion eau-dans-l'huile, une émulsion huile-dans-l'eau, ou un gel.

23. Composition selon l'une quelconque des revendications précédentes, caractérisée par le fait qu'elle contient :

- de 10 à 100 % en poids de ladite dispersion ;
- de 0 à 90 % en poids d'eau ;
- de 0 à 10 % en poids de tensioactifs ;
- de 0 à 40 % en poids d'autres ingrédients actifs ou additifs usuels non particulaires.

24. Utilisation, dans la préparation d'une composition cosmétique capable d'estomper les défauts de la peau tout en laissant sur celle-ci une couche translucide, d'une dispersion, dans un liant gras, d'une charge constituée de particules solides d'au moins un matériau compatible avec l'application sur la peau, ledit liant gras comprenant une fraction non volatile et éventuellement une fraction volatile, ladite fraction volatile représentant, lorsqu'elle est présente, moins de 20 % du poids total de la composition, ladite dispersion contenant, éventuellement, à l'état dissous dans ledit liant gras, des ingrédients oléosolubles, caractérisée par le fait :

- que ladite charge contient au moins 50 % en poids, ou au moins 75 % en volume, par rapport au poids total de la charge, de particules sphériques ou sphéroïdales avant des dimensions de 0,5 à 50 µm,
- et que la concentration volumique C (en %) de ladite charge, dans ladite dispersion, est au moins égale à C*, étant entendu que C est égale à :

$$\frac{V}{V+V1} \times 100$$

V étant le volume de la charge, et V1 étant le volume de la fraction non volatile du liant gras contenant à l'état dissous les ingrédients oléosolubles éventuellement présents dans ladite composition,
et étant entendu que C*, exprimée en %, est comprise entre 3 % et 90 % et est égale à :

$$\frac{V}{V+Vh} \times 100$$

V étant le volume de la charge et Vh représentant la prise d'huile de ladite charge, ladite prise d'huile étant mesurée avec ladite fraction non volatile du liant gras contenant éventuellement, à l'état dissous, lesdits ingrédients oléosolubles, lorsqu'ils sont présents dans ladite composition.

25. Utilisation selon la revendication précédente, caractérisée par le fait que ladite dispersion est telle que définie dans l'une quelconque des revendications 2 à 21.

26. Procédé de préparation d'une composition cosmétique capable d'estomper les défauts de la peau tout en laissant sur celle-ci une couche translucide, ladite composition comprenant une dispersion, dans un liant gras, d'une charge constituée de particules solides d'au moins un matériau compatible avec l'application sur la peau, ledit liant gras comprenant une fraction non volatile et éventuellement une fraction volatile, ladite fraction volatile représentant, lorsqu'elle est présente, moins de 20 % du poids total de la composition, et éventuellement des ingrédients oléosolubles dissous dans le liant gras, caractérisé par le fait que l'on choisit une charge contenant au moins 50 % en poids, ou au moins 75 % en volume, par rapport au poids total de la charge, de particules sphériques ou sphéroïdales ayant des dimensions de 0,5 à 50 µm,

- et que la concentration volumique C (en %) de ladite charge, dans ladite dispersion, est au moins égale à C*, étant entendu que C est égale à :

$$\frac{V}{V+V1} \times 100$$

V étant le volume de la charge, et V1 étant le volume de la fraction non volatile du liant gras contenant à l'état dissous les ingrédients oléosolubles éventuellement présents dans ladite composition,
et étant entendu que C*, exprimée en %, est comprise entre 3 % et 90 % et est égale à :

$$\frac{V}{V+Vh} \times 100$$

V étant le volume de la charge et Vh représentant la prise d'huile de ladite charge, ladite prise d'huile étant mesurée avec ladite fraction non volatile du liant gras contenant éventuellement, à l'état dissous, lesdits ingrédients oléosolubles, lorsqu'ils sont présents dans ladite composition.

**27.** Procédé selon la revendication précédente, caractérisé par le fait que ladite dispersion est telle que définie dans l'une quelconque des revendications 2 à 21.

**28.** Procédé de traitement cosmétique permettant notamment d'estomper les défauts de la peau, caractérisé par le fait que l'on applique sur les zones de peau à traiter une quantité efficace d'une composition telle que définie dans l'une quelconque des revendications 1 à 23.

**Claims**

**1.** Cosmetic composition capable of attenuating skin defects while at the same time leaving a translucent layer thereon, the said composition comprising:

- a dispersion, in a fatty binder, of a charge consisting of solid particles of at least one material which is compatible with application to the skin, the said fatty binder comprising a non-volatile fraction and optionally a volatile fraction, the said volatile fraction representing, when it is present, less than 20% of the total weight of the composition,
- and optionally oleosoluble ingredients, which are dissolved in the said fatty binder, characterized in that:
- the said charge contains at least 50% by weight, or at least 75% by volume, relative to the total weight of the charge, of spherical or spheroidal particles 0.5 to 50 µm in size,
- and in that the concentration by volume C (in %) of the said charge, in the said dispersion, is at least equal to C*, it being understood that C is equal to:

$$\frac{V}{V+V1} \times 100$$

V being the volume of the charge and V1 being the volume of the non-volatile fraction of the fatty binder containing, in the dissolved state, the oleosoluble ingredients which may be present in the said composition,
and it being understood that C*, expressed in %, is between 3% and 90% and is equal to:

$$\frac{V}{V+Vh} \times 100$$

V being the volume of the charge and Vh representing the oil uptake of the said charge, the said oil uptake being measured with the said non-volatile fraction of the fatty binder optionally containing, in the dissolved state, the said oleosoluble ingredients, when they are present in the said composition.

**2.** Composition according to Claim 1, characterized in that the said charge contains at least 75 % by weight, or at least 80 % by volume, of the said spherical or spheroidal particles relative to the total charge.

**3.** Composition according to Claim 1, characterized in that the said charge contains at least 85% by weight, or at least 90% by volume, of the said spherical or spheroidal particles relative to the total charge.

**4.** Composition according to any one of the preceding claims, characterized in that the said spherical or spheroidal particles are from 0.5 to 25 µm in size.

**5.** Composition according to the preceding claim, characterized in that the said spherical or spheroidal particles may range from 1 to 15 µm in size.

**6.** Composition according to any one of the preceding claims, characterized in that the said concentration by volume C* is between 4 and 80%.

7. Composition according to any one of the preceding claims, characterized in that the ratio of the said concentration by volume C to the concentration C* is greater than 1.

8. Composition according to the preceding claim, characterized in that the said ratio is greater than or equal to 1.1.

9. Composition according to the preceding claim, characterized in that the said ratio is high enough for the light transmission, measured between two quartz slides over an optical path length of 100 μm, of the said dispersion (containing all the ingredients of this dispersion except for the volatile oils which may be present) to be at least equal to 5%, at a wavelength of 560 nm.

10. Composition according to any one of the preceding claims, characterized in that, with the exception of the pigments which may be present, the said charge consists solely of the said spherical or spheroidal particles.

11. Composition according to any one of Claims 1 to 6, characterized in that the said charge also contains a minor amount of particles of lamellar form.

12. Composition according to any one of the preceding claims, characterized in that the said material is an inorganic material, an organic material of natural origin or a synthetic polymer.

13. Composition according to any one of the preceding claims, characterized in that the said material is chosen from silica, titanium dioxide, silicone resins, starch, polyamides, polyethylene, poly(methacrylic acids), optionally crosslinked polystyrene, poly(beta-alanine) and polytetrafluoroethylene.

14. Composition according to any one of the preceding claims, characterized in that the said charge also comprises white or coloured pigments.

15. Composition according to the preceding claim, characterized in that the said white or coloured pigments have particle sizes of less than 1 μm, and in particular of less than 0.5 μm.

16. Composition according to either of Claims 14 and 15, characterized in that the said white or coloured pigments are present at a proportion of less than 25% by weight, and in particular of less than 15%, relative to the total charge.

17. Composition according to any one of Claims 1 to 13, characterized in that it is free of white or coloured pigments.

18. Composition according to any one of the preceding claims, characterized in that the binder is an animal, plant or synthetic oil, a mixture of oils or a mixture of oil(s) and wax(es).

19. Composition according to any one of the preceding claims, characterized in that the non-volatile fraction of the said fatty binder has a melting point or a softening point of not more than 37°C.

20. Composition according to any one of the preceding claims, characterized in that the non-volatile fraction of the said fatty binder has a melting point or a softening point of not more than 32°C.

21. Composition according to any one of the preceding claims, characterized in that the non-volatile fraction of the fatty binder has a light transmission at 560 nm of at least 90% over an optical path length of 1 cm.

22. Composition according to any one of the preceding claims, characterized in that the said dispersion is incorporated into a cosmetic vehicle so as to constitute a water-in-oil emulsion, an oil-in-water emulsion or a gel.

23. Composition according to any one of the preceding claims, characterized in that it contains:

   - from 10 to 100% by weight of the said dispersion;
   - from 0 to 90% by weight of water;
   - from 0 to 10% by weight of surfactants;
   - from 0 to 40% by weight of other common, non-particulate additives or active ingredients.

24. Use, in the preparation of a cosmetic composition capable of attenuating skin defects while at the same time leaving a translucent layer thereon, of a dispersion, in a fatty binder, of a charge consisting of solid particles of at least one material which is compatible with application to the skin, the said fatty binder comprising a non-volatile fraction and optionally a volatile fraction, the said volatile fraction representing, when it is present, less than 20% of the total weight of the composition, the said dispersion optionally containing, in the dissolved state in the said fatty binder, oleosoluble ingredients, characterized in that:

- the said charge contains at least 50% by weight, or at least 75% by volume, relative to the total weight of the charge, of spherical or spheroidal particles 0.5 to 50 μm in size,
- and in that the concentration by volume C (in %) of the said charge, in the said dispersion, is at least equal to C*, it being understood that C is equal to:

$$\frac{V}{V+V1} \times 100$$

V being the volume of the charge and V1 being the volume of the non-volatile fraction of the fatty binder containing, in the dissolved state, the oleosoluble ingredients which may be present in the said composition, and it being understood that C*, expressed in %, is between 3% and 90% and is equal to:

$$\frac{V}{V+Vh} \times 100$$

V being the volume of the charge and Vh representing the oil uptake of the said charge, the said oil uptake being measured with the said non-volatile fraction of the fatty binder optionally containing, in the dissolved state, the said oleosoluble ingredients, when they are present in the said composition.

25. Use according to the preceding claim, characterized in that the said dispersion is as defined in any one of Claims 2 to 21.

26. Process for the preparation of a cosmetic composition capable of attenuating skin defects while at the same time leaving a translucent layer thereon, the said composition comprising a dispersion, in a fatty binder, of a charge consisting of solid particles of at least one material which is compatible with application to the skin, the said fatty binder comprising a non-volatile fraction and optionally a volatile fraction, the said volatile fraction representing, when it is present, less than 20% of the total weight of the composition, and optionally oleosoluble ingredients, which are dissolved in the fatty binder, characterized in that a charge is chosen which contains at least 50 % by weight, or at least 75 % by volume, relative to the total weight of the charge, of spherical or spheroidal particles 0.5 to 50 μm in size,

- and in that the concentration by volume C (in %) of the said charge, in the said dispersion, is at least equal to C*, it being understood that C is equal to:

$$\frac{V}{V+V1} \times 100$$

V being the volume of the charge and V1 being the volume of the non-volatile fraction of the fatty binder containing, in the dissolved state, the oleosoluble ingredients which may be present in the said composition, and it being understood that C*, expressed in %, is between 3% and 90% and is equal to:

$$\frac{V}{V+Vh} \times 100$$

V being the volume of the charge and Vh representing the oil uptake of the said charge, the said oil uptake being measured with the said non-volatile fraction of the fatty binder optionally containing, in the dissolved state, the said oleosoluble ingredients, when they are present in the said composition.

27. Process according to the preceding claim, characterized in that the said dispersion is as defined in any one of Claims 2 to 21.

28. Cosmetic treatment process which makes it possible in particular to attenuate skin defects, characterized in that an effective amount of a composition as defined in any one of claims 1 to 23 is applied to the areas of skin to be treated.

**Patentansprüche**

1. Kosmetische Zubereitung mit einer Fähigkeit zum Auswischen von Hautdefekten durch das Stehenlassen einer durchsichtigen Schicht auf der Haut, wobei die Zusammensetzung die folgenden Bestandteile aufweist: eine Dispersion, einen Träger in einem fettigen Bindemittel, der aus festen Teilchen aus mindestens einem mit der Hautapplikation verträglichen Material besteht, wobei das fettige Bindemittel eine nichtflüchtige und gegebenenfalls auch noch eine flüchtige Fraktion enthält und die flüchtige Fraktion im Fall ihrer Anwesenheit weniger als 20 Gew.-% in bezug auf die gesamte Zubereitung ausmacht, und gegebenenfalls öllösliche, in dem fettigen Bindemittel aufgelöste Zusatzstoffe,

dadurch gekennzeichnet, daß

der Träger mindestens 50 Gew.-% oder mindestens 75 Vol.-% kugelige oder kugelähnliche Teilchen mit Abmessungen von 0,5 bis 50 µm in bezug auf das Gesamtgewicht des Trägers enthält, und

die Volumenkonzentration C (in %) des Trägers in der Dispersion mindestens oder gleich C* beträgt,

wobei die Maßgabe gilt, daß C gleich

$$\frac{V}{V+V1} \times 100$$

ist,

wobei V das Trägervolumen und VI das Volumen der nichtflüchtigen Fraktion des fettigen Bindemittels darstellen, welches die gegebenenfalls in der Zusammensetzung vorhandenen öllöslichen Zusatzstoffe in gelöstem Zustand enthält,

sowie die Maßgabe gilt, daß C*, ausgedrückt in %, zwischen 3 % und 90 % und gleich

$$\frac{V}{V+Vh} \times 100$$

ist,

wobei V das Trägervolumen und Vh die kleine Menge an Öl in dem Träger bedeuten, wobei die kleine Menge an Öl mit der nichtflüchtigen Fraktion des fettigen Bindemittels mit einem eventuellen Gehalt an öllöslichen Zusatzstoffen in gelöstem Zustand gemessen wird, sofern die öllöslichen Bestandteile in der Zubereitung vorhanden sind.

2. Zubereitung nach Anspruch 1, dadurch gekennzeichnet, daß der Träger mindestens 75 Gew.-% oder mindestens 80 Vol.-% an kugeligen oder kugelähnlichen Teilchen in bezug auf dessen Gesamtmenge enthält.

3. Zubereitung nach Anspruch 1, dadurch gekennzeichnet, daß der Träger mindestens 85 Gew.-% oder mindestens 90 Vol.-% an kugeligen oder kugelähnlichen Teilchen in bezug auf dessen Gesamtmenge enthält.

4. Zubereitung nach einem der vorstehenden Ansprüche, dadurch gekennzeichnet, daß die kugeligen oder kugelähnlichen Teilchen Abmessungen von 0,5 bis 25 µm aufweisen.

5. Zubereitung nach dem vorstehenden Anspruch 1, dadurch gekennzeichnet, daß die kugeligen oder kugelähnlichen Teilchen Abmessungen aufweisen, die von 1 bis 15 µm gehen können.

6. Zubereitung nach einem der vorstehenden Ansprüche, dadurch gekennzeichnet, daß die Volumenkonzentration C* zwischen 4 und 80 % beträgt.

7. Zubereitung nach einem der vorstehenden Ansprüche, dadurch gekennzeichnet, daß das Verhältnis der Volumenkonzentration C zur Konzentration C* größer als 1 ist.

8. Zubereitung nach dem vorstehenden Anspruch, dadurch gekennzeichnet, daß das Verhältnis größer oder gleich 1,1 ist.

9. Zubereitung nach dem vorstehenden Anspruch, dadurch gekennzeichnet, daß das Verhältnis ausreichend groß ist für die Transmission des Lichts, welche zwischen zwei Quarzplättchen mit einer Lichtstrecke von 100 µm gemessen wird, und zwar in bezug auf die Dispersion mit einem Gehalt an allen Zusatzstoffen darin mit Ausnahme der gegebenenfalls vorhandenen flüchtigen Öle, wobei die Transmission mindestens gleich 5 % bei einer Wellenlänge von 560 nm beträgt.

10. Zubereitung nach einem der vorstehenden Ansprüche, dadurch gekennzeichnet, daß der Träger mit Ausnahme der gegebenenfalls vorhandenen Pigmente einzig und allein aus den kugeligen oder kugelähnlichen Teilchen besteht.

11. Zubereitung nach einem der Ansprüche 1 bis 6, dadurch gekennzeichnet, daß der Träger zusätzlich noch eine kleinere Menge an blättchenförmigen Teilchen enthält.

12. Zubereitung nach einem der vorstehenden Ansprüche, dadurch gekennzeichnet, daß das Material ein mineralisches oder organisches Material natürlichen Ursprungs oder ein synthetisches Polymer darstellt.

13. Zubereitung nach einem der vorstehenden Ansprüche, dadurch gekennzeichnet, daß das Material aus Kieselerde, Titandioxid, Silikonharzen, Stärke, Polyamiden, Polyethylen, Polymethacrylsäuren, gegebenenfalls vernetztem Polystyrol, Polyβ-alanin und Polytetrafluorethylen ausgewählt ist.

14. Zubereitung nach einem der vorstehenden Ansprüche, dadurch gekennzeichnet, daß der Träger zusätzlich noch weiße oder farbige Pigmente enthält.

15. Zubereitung nach dem vorstehenden Anspruch, dadurch gekennzeichnet, daß die weißen oder farbigen Pigmente Teilchenabmessungen von kleiner als 1 μm, insbesondere unterhalb von 0,5 μm, besitzen.

16. Zubereitung nach einem der Ansprüche 14 und 15, dadurch gekennzeichnet, daß die weißen oder farbigen Pigmente in einem Verhältnis von mindestens 25 Gew.-%, insbesondere weniger als 15 Gew.-%, in bezug auf das Gesamtgewicht des Trägers vorhanden sind.

17. Zubereitung nach einem der Ansprüche 1 bis 13, dadurch gekennzeichnet, daß sie frei ist von weißen oder farbigen Pigmenten.

18. Zubereitung nach einem der vorstehenden Ansprüche, dadurch gekennzeichnet, daß das Bindemittel ein tierisches, pflanzliches oder synthetisches Öl, ein Gemisch aus Ölen oder ein Gemisch aus Öl(en) und Wachs(en) darstellt.

19. Zubereitung nach einem der vorstehenden Ansprüche, dadurch gekennzeichnet, daß die nichtflüchtige Fraktion des fettigen Bindemittels einen Schmelzpunkt oder Erweichungspunkt von nicht mehr als 37 °C aufweist.

20. Zubereitung nach einem der vorstehenden Ansprüche, dadurch gekennzeichnet, daß die nichtflüchtige Fraktion des fettigen Bindemittels einen Schmelzpunkt oder Erweichungspunkt von nicht mehr als 32 °C aufweist.

21. Zubereitung nach einem der vorstehenden Ansprüche, dadurch gekennzeichnet, daß die nichtflüchtige Fraktion des fettigen Bindemittels eine Lichtdurchlässigkeit von mindestens 90 % über eine Lichtstrecke von 1 cm bei 560 nm besitzt.

22. Zubereitung nach einem der vorstehenden Ansprüche, dadurch gekennzeichnet, daß die Dispersion in einen kosmetischen Träger in der Weise eingearbeitet ist, daß die Emulsion eine Wasser-in-Öl- oder eine Öl-in-Wasser-Emulsion oder ein Gel bildet.

23. Zubereitung nach einem der vorstehenden Ansprüche, dadurch gekennzeichnet, daß sie

- 10 bis 100 Gew.-% Dispersion,
- 0 bis 90 Gew.-% Wasser,
- 0 bis 10 Gew.-% Tenside, sowie
- 0 bis 40 Gew.-% anderer Wirkstoffe oder üblicher nichtteilchenförmiger Zusatzstoffe

enthält.

24. Verwendung einer Dispersion in einem fettigen Bindemittel sowie eines Trägers bei der Herstellung einer kosmetischen Zusammensetzung mit der Fähigkeit, Hautdefekte zu verwischen, indem auf ihr eine durchsichtige Schicht zurückbleibt, wobei der Träger aus festen Teilchen mindestens eines Materials besteht, das mit der Applikation auf der Haut verträglich ist, und wobei das fettige Bindemittel eine nichtflüchtige und gegebenenfalls noch eine flüchtige Fraktion enthält und die flüchtige Fraktion im Falle ihrer Anwesenheit weniger als 20 Gew.-% in bezug auf die gesamte Zubereitung ausmacht, sowie gegebenenfalls öllösliche, in dem fettigen Bindemittel aufgelöste Zusatzstoffe,
dadurch gekennzeichnet, daß
der Träger mindestens 50 Gew.-% oder mindestens 75 Vol.-% kugelige oder kugelähnliche Teilchen mit Abmessungen von 0,5 bis 50 μm in bezug auf das Gesamtgewicht des Trägers enthält, und
die Volumenkonzentration C (in %) des Trägers in der Dispersion mindestens oder gleich C* beträgt,
wobei die Maßgabe gilt, daß C gleich

$$\frac{V}{V+V1} \times 100$$

ist,

wobei V das Trägervolumen und VI das Volumen der nichtflüchtigen Fraktion des fettigen Bindemittels darstellen, welches die gegebenenfalls in der Zusammensetzung vorhandenen öllöslichen Zusatzstoffe in gelöstem Zustand enthält,
sowie die Maßgabe gilt, daß C*, ausgedrückt in %, zwischen 3 % und 90 % und gleich

$$\frac{V}{V+Vh} \times 100$$

ist,

wobei V das Trägervolumen und Vh die kleine Menge an Öl in dem Träger bedeuten, wobei die kleine Menge an Öl mit der nichtflüchtigen Fraktion des fettigen Bindemittels mit einem eventuellen Gehalt an öl löslichen Zusatzstoffen in gelöstem Zustand gemessen wird, sofern die öllöslichen Bestandteile in der Zubereitung vorhanden sind.

25. Verwendung nach dem vorangehenden Anspruch, dadurch gekennzeichnet, daß die Dispersion der Definition gemäß einem der Ansprüche 2 bis 21 entspricht.

26. Verfahren zur Herstellung einer kosmetischen Zubereitung mit der Fähigkeit zum Auswischen von Hautdefekten durch das Stehenlassen einer durchsichtigen Schicht auf der Haut, wobei die Zusammensetzung die folgenden Bestandteile aufweist:
eine Dispersion, einen Träger in einem fettigen Bindemittel, der aus festen Teilchen aus mindestens einem mit der Hautapplikation verträglichen Material besteht, wobei das fettige Bindemittel eine nichtflüchtige und gegebenenfalls noch eine flüchtige Fraktion enthält und die flüchtige Fraktion im Fall ihrer Anwesenheit weniger als 20 Gew.-% in bezug auf die gesamte Zubereitung ausmacht, und gegebenenfalls öllösliche, in dem fettigen Bindemittel aufgelöste Zusatzstoffe,
dadurch gekennzeichnet, daß
ein Träger mit einem Gehalt an mindestens 50 Gew.-% oder mindestens 75 Vol.-% und kugelige oder kugelähnliche Teilchen mit Abmessungen von 0,5 bis 50 µm in bezug auf das Gesamtgewicht des Trägers ausgewählt werden, die Volumenkonzentration C (in %) des Trägers in der Dispersion mindestens oder gleich C* beträgt,
wobei die Maßgabe gilt, daß C gleich

$$\frac{V}{V+V1} \times 100$$

ist,

wobei V das Trägervolumen und VI das Volumen der nichtflüchtigen Fraktion des fettigen Bindemittels darstellen, welches die gegebenenfalls in der Zusammensetzung vorhandenen öllöslichen Zusatzstoffe in gelöstem Zustand enthält,
sowie die Maßgabe gilt, daß C*, ausgedrückt in %, zwischen 3 % und 90 % und gleich

$$\frac{V}{V+Vh} \times 100$$

ist,

wobei V das Trägervolumen und Vh die kleine Menge an Öl in dem Träger bedeuten, wobei die kleine Menge an Öl mit der nichtflüchtigen Fraktion des fettigen Bindemittels mit einem eventuellen Gehalt an öllöslichen Zusatzstoffen in gelöstem Zustand gemessen wird, sofern die öllöslichen Bestandteile in der Zubereitung vorhanden sind.

27. Verfahren nach dem vorstehenden Anspruch, dadurch gekennzeichnet, daß die Dispersion der in einem der Ansprüche 2 bis 21 gegebenen Definition entspricht.

28. Verfahren zur kosmetischen Behandlung, insbesondere mit der Eigenschaft, Hautdefekte zu verwischen, dadurch gekennzeichnet, daß auf die zu behandelnden Hautzonen eine ausreichende Menge an einer Zubereitung gemäß Definition in einem der Ansprüche 1 bis 23 aufgebracht wird.